# EUROPÄISCHE PATENTANMELDUNG

(11) **EP 3 766 338 A1**
(43) Veröffentlichungstag der Anmeldung: **20.01.2021**
(21) Anmeldenummer: 19186463.6
(22) Anmeldetag: 16.07.2019
(51) Int. Cl.: A01G 25/16, G01N 33/24, G01N 21/55

(54) **OPTISCHES TENSIOMETER**

(71) Anmelder: Meter Group AG, 81379 München (DE)
(72) Erfinder: HIRTE, Josef, 80799 München (DE)
(74) Vertreter: Lambacher, Michael

(57) **Zusammenfassung**

Bereitgestellt wird ein optischer Sensor (10) zur Erfassung von Strahlungsintensitäten, die auf ein Matrixpotential in einem den Sensor (10) umgebenden Medium (20) hinweisen. Der Sensor (10) umfasst ein semipermeables Messplättchen (12), das eine Innenfläche (12a) und eine Außenfläche (12b) aufweist, wobei die Außenfläche (12b) dazu ausgebildet ist, mit dem Medium (20) in Kontakt zu treten. Ferner umfasst der optische Sensor einen Strahlungssender (14), der dazu ausgebildet ist, Strahlung (15) auf die Innenfläche (12a) des Messplättchens (12) zu strahlen; und einen Strahlungsempfänger (16), der dazu ausgebildet ist, die von der Innenfläche (12a) des Messplättchens (12) reflektierte Strahlung (15) zu empfangen. Das Messplättchen (12) ist aus einem porösen photokatalytisch aktiven Material aufgebaut und die Strahlung (15) ist dazu konfiguriert, das photokatalytisch aktive Material anzuregen. Ferner werden eine Messsonde umfassend wenigstens einen erfindungsgemäßen Sensor, ein Verfahren zur Messung eines Matrixpotentials mittels des erfindungsgemäßen Sensors, sowie die Verwendung eines photokatalytisch aktiven Materials und einer das photokatalytische Material anregenden Strahlung zur Messung eines Matrixpotentials bereitgestellt.

## Beschreibung

### Gebiet der Technik

Die Erfindung betrifft allgemein das Messen von Matrixpotential in Medien wie beispielsweise Erdböden. Insbesondere betrifft die Erfindung einen optischen Sensor zur Erfassung von Strahlungsintensitäten, die auf ein Matrixpotential in einem den Sensor umgebenden Medium hinweisen, sowie eine Messsonde, in welcher der erfindungsgemäße Sensor verbaut ist.

### Stand der Technik

Der Begriff Matrixpotential entspricht in seiner Bedeutung den Begriffen Bodenwasserspannung, Saugspannung und Wasserspannung. Das Matrixpotential ist ein wichtiger Parameter zur Charakterisierung des Bodenwasserhaushalts. Insbesondere ist das Matrixpotential ausschlaggebend für das Pflanzenwachstum, da es die Verfügbarkeit des Wassers für Pflanzen direkt angibt. Daneben ist die Bestimmung des Matrixpotentials in Lebensmitteln von Bedeutung, da alle mikrobiellen Prozesse ebenfalls von diesem Parameter abhängig sind.

Zur direkten Messung des Matrixpotentials werden bisher vorzugsweise Tensiometer eingesetzt. Sie bestehen aus einem Hohlraum, der vollständig mit Wasser gefüllt ist. Dieser steht mit dem Boden über eine semipermeable Membran (vorzugsweise eine feinporöse Keramik) in Verbindung, die wasserdurchlässig ist und Gase sperrt. Die Membran überträgt die Bodenwasserspannung über den Kapillarenverbund (offenporige Struktur der Membran) auf das Tensiometerwasser, wodurch ein messbarer atmosphärischer Unterdruck entsteht. Dieser kann zum Beispiel mithilfe eines elektronischen Drucksensors gemessen und angezeigt werden.

Solche Tensiometer messen jedoch nur in einem eingeschränkten Messbereich (0 bis ca. 0,9 bar), der durch den Dampfpunkt des Wassers begrenzt und damit wesentlich kleiner ist als der pflanzenphysiologisch relevante Messbereich (0 bis ca. 15 bar). Ab Erreichen des Dampfpunktes verdampft das Wasser, wodurch das Tensiometer die Bodenwasserspannung nicht mehr auf den Druckaufnehmer übertragen kann und "leer läuft". Das Wasser wird aus dem Tensiometer herausgesaugt. Ein leer gelaufenes Tensiometer bedeutet jedoch einen erhöhten Wartungsaufwand, da es neu befüllt werden muss.

Aus der DE 100 47 937 C1 ist eine Technik zur Messung des Matrixpotentials in Böden mittels optischer Abtastung eines porösen, hydrophilen Materials bekannt. Dabei werden die durch die sich ändernde Bodenwasserspannung hervorgerufenen Änderungen der Reflexionseingenschaften des porösen Materials optisch vermessen. Um eine langzeitstabile Messung durchführen zu können, muss das poröse Material auf der Innenseite, die optisch abgetastet wird, vor Befall bzw. Ansiedlung von Mikroorganismen geschützt werden. Nach den Lehren der DE 100 47 937 C1 kann dazu die Innenseite mit einem Fungizid behandelt, oder eine Silberschicht in das poröse Material integriert werden. Dieser Ansatz ist aufwändig.

Eine Aufgabe der vorliegenden Erfindung besteht deshalb darin, eine alternative Lösung zum Schutz des porösen Materials vor dem Befall bzw. der Ansiedlung von Mikroorganismen an der Innenfläche des porösen Materials bereitzustellen.

### Kurzer Abriss der Erfindung

Zur Lösung der der Erfindung zugrundeliegenden Aufgabe wird ein optischer Sensor bereitgestellt, der der Erfassung von Strahlungsintensitäten dient, die auf ein Matrixpotential in einem den Sensor umgebenden Medium hinweisen. Der Sensor umfasst ein semipermeables Messplättchen, das eine Innenfläche und eine Außenfläche aufweist, wobei die Außenfläche dazu ausgebildet ist, mit dem Medium in Kontakt zu treten. Ferner umfasst der Sensor einen Strahlungssender, der dazu ausgebildet ist, Strahlung auf die Innenfläche des Messplättchens zu strahlen, und einen Strahlungsempfänger, der dazu ausgebildet ist, die von der Innenseite des Messplättchens reflektierte Strahlung zu empfangen. Das Messplättchen ist aus einem porösen photokatalytisch aktiven Material aufgebaut und die von dem Strahlungssender emittierte Strahlung ist dazu konfiguriert, das photokatalytisch aktive Material anzuregen.

Das den Sensor umgebende Medium kann Erde sein. Die Messung von Matrixpotential in Erdböden kann insbesondere im landwirtschaftlichen Bereich von Relevanz sein.

Der Begriff "semipermeables Messplättchen" ist im Rahmen der vorliegenden Erfindung so auszulegen, dass das Messplättchen wasserdurchlässig und gasundurchlässig ist. Diese Semipermeabilität wird erreicht durch eine feinporöse Struktur des Messplättchens. Das poröse Material, aus dem das Messplättchen aufgebaut ist, weist eine offenporige Struktur auf. Durch die miteinander in Verbindung stehenden Poren entsteht ein Netzwerk feiner Kanäle die sich zwischen der Außenfläche und der Innenfläche über die gesamte Dicke des Messplättchens erstrecken. Die feinporige Struktur des Messplättchens ruft bei dem Kontakt mit Flüssigkeit einen Kapillareffekt hervor. Kommt die Außenfläche des Messplättchens mit einem (feuchten) Medium - z.B. Erde im Erdboden - in Kontakt, so füllen sich die Poren des Messplättchens mit Wasser.

Abhängig von dem Matrixpotential in dem mit der Außenfläche des Messplättchens in Kontakt stehenden Mediums weist der Wasserspiegel, der jeweils an den Poren der Innenfläche des Messplättchens ausgebildet ist, einen unterschiedlichen Wölbungsgrad auf. Bei einem geringen Matrixpotential etwa (gesättigtes Medium, feuchter Boden), weisen die Poren an der Innenfläche des Messplättchens jeweils konvex gewölbte Wasserspiegel auf. Mit zunehmendem Matrixpotential (ungesättigtes Medium, trockener Boden), zieht sich an der Innenfläche des Messplättchens der Wasserspiegel in jeder Pore zurück und formt jeweils eine konkave Wölbung.

Abhängig von dem Wölbungsgrad des Wasserspiegels in den Poren an der Innenfläche des Messplättchens, ändern sich die Reflexionseigenschaften der Innenfläche. Der Strahlungssender strahlt die Strahlung in einem vorbestimmten Einstrahlwinkel (bzw. Einfallwinkel) auf die Innenfläche des Messplättchens. Der Strahlungsempfänger empfängt die von der Innenfläche reflektierte Strahlung in einem auf den Einstrahlwinkel abgestimmten Reflexionswinkel. Bei einer Änderung der Reflexionseigenschaften der Innenfläche des Messplättchens ändert sich auch die Intensität der von dem Strahlungsempfänger empfangenen Strahlung, da durch Streuung nur noch ein Teil der Strahlung in Richtung des Reflexionswinkels reflektiert wird. Da die Reflexionseigenschaften der Innenfläche von dem Matrixpotential des anliegenden Mediums abhängen, steht die Intensitätsänderung der empfangenen Strahlung in direktem Zusammenhang mit der Änderung des Matrixpotentials.

Gemäß einer Variante kann die Strahlung eine Wellenlänge im UV-Bereich aufweisen. Mit anderen Worten kann die Strahlung eine Wellenlänge zwischen 100 nm und 400 nm aufweisen. Gemäß einer bevorzugten Variante kann die Strahlung eine Wellenlänge zwischen 380 nm und 390 nm, insbesondere von 385 nm aufweisen. Strahlung dieser Wellenlänge hat sich bei Versuchen als besonders vorteilhaft herausgestellt.

Die Kombination des Einsatzes photokatalytisch aktiven Materials und das photokatalytische Material anregender Strahlung (insbesondere UV-Strahlung) ruft an den Stellen, an denen die Innenfläche des Messplättchens bestrahlt wird, einen photokatalytischen Effekt hervor. Der Effekt bewirkt die Zersetzung von Mikroorgansimen an der Innenfläche zu H₂O und CO₂ und somit eine Selbstreinigung der Innenfläche ohne zusätzliche Beschichtung.

Das Material, aus dem das Messplättchen aufgebaut ist, kann eine hydrophile Keramik sein. Ferner kann das Material temperaturstabil und im gesamten pH-Bereich chemisch beständig sein. Beispielsweise kann das Messplättchen aus Titandioxid (TiO₂) aufgebaut sein. Titandioxid verfügt bei Anregung mit ausreichend hoher Energie (etwa durch die Bestrahlung mit UV-Licht) über eine hohe photokatalytische Aktivität und Superhydrophilität. Zudem ist TiO₂ ungiftig und inert und somit für den umweltfreundlichen Einsatz in Erdböden in besonderem Maße geeignet.

Das poröse Material des Messplättchens kann eine Porengröße von weniger als 800 nm aufweisen. Die Porengröße beschreibt den größtmöglichen Durchmesser einer sphärischen Kugel, die den Porenverbund gerade noch passieren kann.

Gemäß einer Variante kann das Messplättchen einen ersten Bereich aufweisen, wobei die Porengröße in dem ersten Bereich zwischen 400 nm und 800 nm liegt. Ein Messplättchen mit dieser Porengröße eignet sich für präzise Messungen von Matrixpotential in einem Messbereich zwischen 0 bar und 1 bar, d.h. für Messungen in einem gesättigten Medium (z.B. einem feuchten Erdboden).

Zusätzlich oder alternativ kann das Messplättchen zur Messung von Matrixpotential in Medien mit geringer Sättigung (z.B. trockene Erdböden) einen zweiten Bereich aufweisen, wobei die Porengröße in dem zweiten Bereich unterhalb von 400 nm liegt. Durch die geringe Porengröße kann eine Entleerung der Poren beim Austrocknen des Messplättchens vermieden werden. Durch die Verwendung einer Porengröße unterhalb von 400 nm kann ein Messbereich bis ca. 15 bar abgedeckt werden.

Es versteht sich, dass das Messplättchen ausschließlich den ersten Bereich oder alternativ ausschließlich den zweiten Bereich aufweisen kann. Gemäß einer weiteren Alternative kann das Messplättchen sowohl den ersten Bereich als auch den zweiten Bereich aufweisen, wodurch der Messbereich des optischen Sensors bei gleichzeitiger hoher Messgenauigkeit vergrößert werden kann.

Der Strahlungsempfänger des optischen Sensors kann dazu ausgebildet sein, eine Intensitätsänderung der empfangenen Strahlung zu erfassen, wobei die Intensitätsänderung auf eine Änderung des Matrixpotentials hinweist.

Sein Aufbau und insbesondere die Anordnung seiner Bestandteile (Komponenten) zueinander ermöglichen eine sehr kompakte Bauweise des Sensors im Vergleich mit herkömmlichen Sensoren. Ein Vorteil einer kompakten Bauweise des Sensors kann in der Eignung zum Einsatz in Laborgeräten bestehen, bei denen eine besonders platzsparende Bauweise erforderlich ist.

Zur Lösung der der Erfindung zugrungeliegenden Aufgabe wird ferner eine Messsonde zur Messung von Matrixpotential in einem die Messsonde umgebenden Medium bereitgestellt. Die Messsonde umfasst einen Korpus und wenigstens einen optischen Sensor gemäß einer der oben beschriebenen Varianten. Der wenigstens eine Sensor ist derart in dem Korpus angeordnet, dass das Messplättchen wenigstens teilweise durch den Korpus hindurchragt. Die Messsonde umfasst ferner eine Steuereinheit, die dazu ausgebildet ist, die Strahlungsintensität und Strahlungsdauer der von dem Strahlungssender emittierten Strahlung zu steuern, und eine Auswerteeinheit, die dazu ausgebildet ist, das Matrixpotential in dem Medium anhand der von dem Strahlungsempfänger empfangenen Strahlungsintensitäten zu berechnen.

Die Steuereinheit und die Auswerteeinheit der Messsonde können jeweils als Mikrokontroller implementiert sein.

Gemäß einer Variante kann der optische Sensor derart in dem Korpus der Messsonde angeordnet sein, dass die Außenfläche des Messplättchens bündig mit einer Außenwand des Korpus abschließt. Dazu kann der Korpus eine Aussparung umfassen, in welcher das Messplättchen angeordnet ist. Durch eine derartige Anordnung des optischen Sensors in der Messsonde kann die Außenfläche des Messplättchens mit dem die Messsonde umgebenden Medium in Kontakt treten. Es ist jedoch auch denkbar, dass die Außenfläche des Messplättchens des optischen Sensors über die Außenwand des Korpus der Messsonde hinausragt oder aber dahinter zurückbleibt und eine Nische in der Außenwand bildet.

Die Messsonde kann ferner ein Schnittstellenmodul umfassen. Das Schnittstellenmodul kann mit der Steuereinheit und/oder der Auswerteeinheit in Verbindung stehen und ist dazu ausgebildet, mit einem externen Rechengerät (z.B. PC, Tablet, oder Smartphone) zu kommunizieren. Zum Beispiel kann das Schnittstellenmodul dazu ausgebildet sein, von dem externen Rechengerät Steuerbefehle zu empfangen und Sensordaten dorthin zu übermitteln.

Gemäß einer Variante kann der Korpus der Messsonde eine zylindrische Form aufweisen. Ferner kann der Korpus einen Durchmesser von weniger als 15 mm aufweisen. Vorzugsweise kann der Durchmesser des Korpus 14 mm betragen. Derartig kleine Durchmesser der Messsonde werden insbesondere durch die kompakte Bauweise des Sensors ermöglicht.

Gemäß einer Variante kann die Messsonde wenigstens zwei der optischen Sensoren umfassen, wobei die wenigstens zwei optischen Sensoren in Längsrichtung des Korpus einen Abstand zueinander aufweisen. Bei der Messung von Matrixpotential im Erdboden können somit gleichzeitig Messwerte in verschiedenen Tiefenbereichen des Bodens aufgenommen und Rückschlüsse auf die Beschaffenheit des Bodens in diesen Tiefenbereichen gezogen werden.

Die Messsonde kann ferner ein Kopfstück aufweisen, welches an einem ersten Längsende des Korpus angebracht ist. Gemäß einer Variante kann das Kopfstück zusammen mit dem Korpus als ein zusammenhängendes Bauteil ausgebildet sein. In radialer Richtung (bezüglich einer Längsachse des Korpus) kann das Kopfstück vorzugsweise eine größere Ausdehnung aufweisen als der Korpus.

An seinem dem ersten Längsende gegenüberliegenden zweiten Längsende kann der Korpus eine Spitze aufweisen. Die Spitze kann beispielsweise durch einen sich verjüngenden Durchmesser des Korpus an dessen zweitem Längsende ausgebildet sein.

Bei der Messung des Matrixpotentials in Erdböden kann die Messsonde mit der Spitze in den Boden getrieben werden. Beispielsweise kann die Messsonde bis zu dem Kopfstück in den Boden getrieben werden, sodass der Korpus vollständig von Erdboden umgeben ist. Gemäß einer Variante kann die Messsonde einen ersten erfindungsgemäßen optischen Sensor aufweisen, der etwa 3 cm bis 7 cm, bevorzugt 5 cm, entfernt von dem Kopfstück in dem Korpus angeordnet ist. Ein zweiter und ein dritter erfindungsgemäßer optischer Sensor können etwa 12 cm bis 18 cm, bevorzugt 15 cm, respektive etwa 35 cm bis 45 cm, bevorzugt 40 cm, entfernt von dem Kopfstück in dem Korpus angeordnet sein. Auf diese Weise kann mittels einer erfindungsgemäßen Messsonde das Matrixpotential in verschiedenen Bodenbereichen gemessen werden. Der Bereich ca. 5 cm unterhalb der Erdoberfläche ist dabei von besonderer Relevanz für die Keimungsphase von Pflanzen. In etwa 15 cm unter der Erdoberfläche befinden sich die Hauptwurzeln vieler Pflanzen. Informationen zu dem Matrixpotential in diesem Tiefenbereich sind daher für die Bestimmung der Wasserversorgungssituation von großer Bedeutung. Die Messung des Matrixpotentials etwa 40 cm unterhalb der Erdoberfläche dient der Überwachung von Überwässerung.

Zur Lösung der der Erfindung zugrundeliegenden Aufgabe wird ferner ein Verfahren zum Messen, mittels eines optischen Sensors gemäß einer der oben beschriebenen Varianten, von Matrixpotential in einem den optischen Sensor umgebenden Medium bereitgestellt.

Das Verfahren umfasst das Bereitstellen des optischen Sensors mit dem Strahlungssender, dem semipermeablen Messplättchen und dem Strahlungsempfänger, sowie das Bereitstellen einer Steuereinheit und einer Auswerteeinheit. Weiter umfasst das Verfahren die folgenden Schritte: Inkontaktbringen der Außenfläche des Messplättchens mit dem den optischen Sensor umgebenden Medium; Strahlen, mittels des Strahlungssenders, von Strahlung auf die Innenfläche des Messplättchens, wobei die Strahlungsintensität und Strahlungsdauer der von dem Strahlungssender emittierten Strahlung durch die Steuereinheit gesteuert wird; Empfangen, mittels des Strahlungsempfängers, der von der Innenfläche des Messplättchens reflektierten Strahlung; und Berechnen, durch die Auswerteeinheit, des Matrixpotentials in dem Medium anhand der Strahlungsintensität der von dem Strahlungsempfänger empfangenen reflektierten Strahlung.

Der Schritt des Empfangens der reflektierten Strahlung kann ferner umfassen: Erfassen einer Intensitätsänderung der empfangenen Strahlung, wobei die Intensitätsänderung auf eine Änderung des Matrixpotentials hinweist.

Zur Lösung der der Erfindung zugrundeliegenden Aufgabe wird außerdem die Verwendung eines photokatalytisch aktiven Materials und einer das photokatalytische Material anregenden Strahlung zur Messung eines Matrixpotentials bereitgestellt.

Die Verwendung des photokatalytisch aktiven Materials und der das photokatalytische Material anregenden Strahlung kann in einem optischen Sensor gemäß einem der oben beschriebenen Varianten implementiert sein, wobei das photokatalytisch aktive Material in Form des Messplättchens von dem optischen Sensors umfasst ist.

### Kurze Figurenbeschreibung

Einzelheiten, Aspekte und Vorteile, die hier beschriebenen Erfindung ergeben sich aus den Nachfolgenden Zeichnungen. Es zeigen:
**Figur 1**: eine Ausführungsform eines erfindungsgemäßen optischen Sensors;
**Figuren 2a****-d**: schematisch den Wasserspiegel in einer Pore eines porösen hydrophilen Materials;
**Figuren 3a****-b**: schematisch die Reflexion von Strahlung in einer planen und einer gewölbten Oberfläche im Vergleich;
**Figur 4**: eine Messsonde gemäß einer Variante der vorliegenden Erfindung; und
**Figur 5**: ein Flussdiagramm eines erfindungsgemäßen Verfahrens zum Messen eines Matrixpotentials.

### Detaillierte Beschreibung

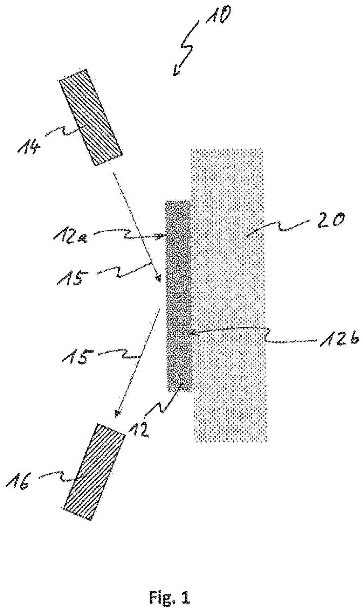

Figur 1 zeigt schematisch den Aufbau eines erfindungsgemäßen optischen Sensors 10 zur Erfassung von Strahlungsintensitäten, die auf ein Matrixpotential in einem den Sensor 10 umgebenden Medium 20 hinweisen.

Der Sensor 10 umfasst ein semipermeables Messplättchen 12, das eine Innenfläche 12a und eine Außenfläche 12b aufweist, wobei die Außenfläche 12b dazu ausgebildet ist, mit dem zu untersuchenden Medium 20 in Kontakt zu treten. Ferner weist der optische Sensor 12 einen Strahlungssender 14 auf. Der Strahlungssender 14 ist dazu ausgebildet, Strahlung 15 unter einem vorbestimmten Einstrahlwinkel (bzw. Einfallwinkel) auf die Innenfläche 12a des Messplättchens 12 zu strahlen. Ein Strahlungsempfänger 16, der ebenfalls von dem optischen Sensor 10 umfasst ist, ist dazu ausgebildet, die von der Innenfläche 12a unter einem vorbestimmten Reflexionswinkel reflektierte Strahlung 15 zu empfangen. Der Reflexionswinkel kann vorzugsweise auf den Einstrahlwinkel abgestimmt sein. Bezogen auf die Innenfläche 12a des Messplättchens kann der Einstrahlwinkel gleich dem Reflexionswinkel sein.

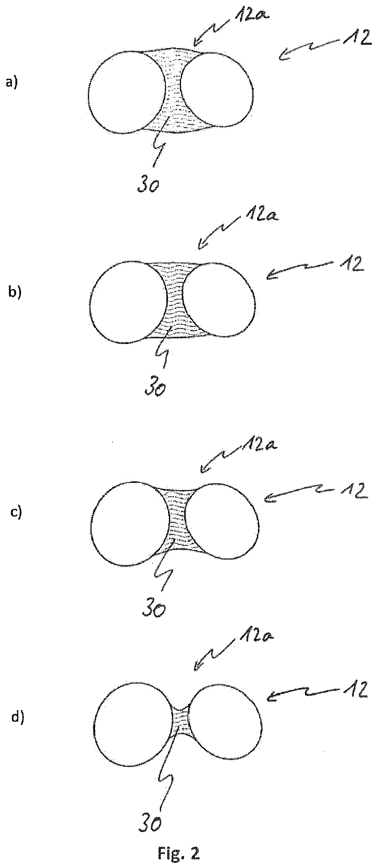

Das Messplättchen 12 ist aus einem porösen photokatalytisch aktiven Material aufgebaut, z.B. Titandioxid. Die Poren in dem Material des Messplättchens 12 bilden einen offenen Porenverbund mit einer Porengröße von maximal 800 nm. Bei Kontakt der Außenfläche 12b mit dem zu untersuchenden Medium 20 (z.B. Erde im Erdboden) füllen sich die Poren des Messplättchens 12 aufgrund von Kapillarität (Kapillareffekt) mit Wasser aus dem Medium. Abhängig von dem Matrixpotential in dem Medium weist der Wasserspiegel, der jeweils an den Poren der Innenfläche 12a des Messplättchens ausgebildet ist, einen unterschiedlichen Wölbungsgrad auf (s. Fig. 2).

Abhängig von dem Wölbungsgrad des Wasserspiegels in den Poren an der Innenfläche 12a des Messplättchens 12, ändern sich die Reflexionseigenschaften der Innenfläche 12a. Mit zunehmender Wölbung des Wasserspiegels steigt auch der Streuungsgrad der an der Innenfläche 12a reflektierten Strahlung. Mit steigender Streuung wiederum sinkt die Intensität der Strahlung 15, die von dem Strahlungsempfänger 16 unter dem Reflexionswinkel empfangen wird.

Wenn also der Strahlungssender 14 unter dem Einstrahlwinkel Strahlung 15 auf die Innenfläche 12a strahlt, ändert sich abhängig von dem Matrixpotential in dem Medium 20 die Intensität der von dem Strahlungsempfänger 16 empfangenen Strahlung 15. Der Strahlungsempfänger 16 kann dazu ausgebildet sein, eine Intensitätsänderung der empfangenen Strahlung 15 zu erfassen. Basierend auf der Intensitätsänderung der empfangenen Strahlung 15 kann direkt die Änderung des Matrixpotentials berechnet werden.

Bei einem geringen Matrixpotential etwa (gesättigtes Medium, feuchter Boden), weisen die Poren an der Innenfläche des Messplättchens jeweils konvex gewölbte Wasserspiegel auf. Mit zunehmendem Matrixpotential (ungesättigtes Medium, trockener Boden), zieht sich an der Innenfläche des Messplättchens der Wasserspiegel in jeder Pore zurück und formt jeweils eine konkave Wölbung.

Durch die Bestrahlung des photokatalytisch aktiven Materials (z.B. Titandioxid) mit UV-Strahlung mit einer Wellenlänge zwischen 100 nm und 400 nm, kommt es zu einer Reaktion an der Innenfläche 12a des Messplättchens 12. Dabei werden Mikroorganismen, die sich als Biofilm an der Innenfläche 12a ansiedeln, zu H₂O und CO₂ zersetzt. Dieser selbstreinigende Prozess trägt dazu bei, dass die Langzeitstabilität von Messungen erhöht werden kann, ohne, dass es einer Beschichtung oder anderer zusätzlicher Maßnahmen zur Reinigung des Messplättchens 12 bedarf.

In Figur 2 sind die Verhältnisse an der Innenfläche 12a des Messplättchens 12 schematisch dargestellt. Gezeigt ist jeweils schematisch eine Pore an der Innenfläche 12a des Messplättchens, wobei die Pore mit Wasser 30 gefüllt ist. Das anliegende Matrixpotential steigt mit den Darstellungen von oben nach unten (d.h. von a) nach d)) an. Die Innenfläche 12a des Messplättchens 12 liegt in den Darstellungen immer oben.

Die erste Darstellung (a)) in Figur 2 zeigt schematisch die Form des Wasserspiegels bei niedrigem anliegendem Matrixpotential. Dieser weist eine konvexe Krümmung auf. Steigt das Matrixpotential an (s. Fig. 2 b) und c)), so nimmt die Wölbung des Wasserspiegels ab und zieht sich in die Pore zurück. Bei hohem anliegendem Matrixpotential krümmt sich der Wasserspiegel konkav in die Pore hinein (s. Fig. 2 d)).

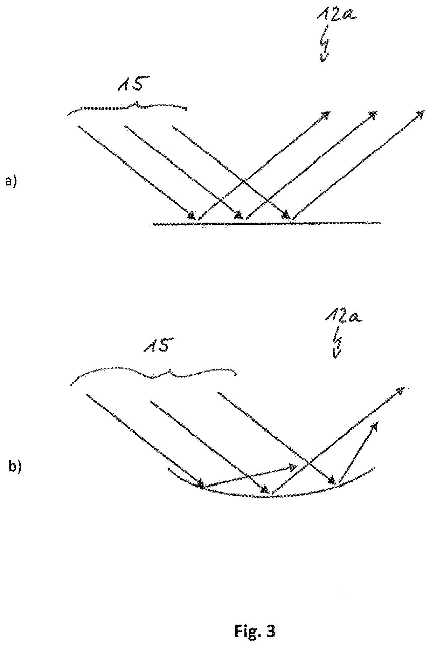

Anhand der Darstellung in Figur 3 wird das Verhalten der Strahlung 15 bei Reflexion an einem nicht gewölbten und einem gewölbten Wasserspiegel in einer Pore an der Innenseite 12a des Messplättchens 12 beschrieben. An dem ebenen Wasserspiegel (s. Fig. 3 a) werden parallele Strahlen wieder parallel in gleichem Winkel reflektiert. Wenn der Strahlungsempfänger 16 (s. Fig. 1) in dem reflektierten Strahlenbündel positioniert ist, entspricht die Intensität der empfangenen Strahlung im Wesentlichen der Intensität der von dem Strahlungssender 14 ausgesandten Strahlung vor der Reflexion. Trifft parallele Strahlung 15 auf einen gewölbten Wasserspiegel (s. Fig. 3 b)), so ergibt sich eine Streuung der reflektierten Strahlung. Je stärker die Wölbung ist, desto mehr wird das Strahlenbündel 15 infolge der Reflexion aufgeweitet. Dabei ist die Richtung der Wölbung (konkav oder konvex) in erster Näherung ohne Bedeutung. Aufgrund der Streuung empfängt der Strahlungsempfänger 16 nur einen Teil der ursprünglich ausgesandten Strahlung 15. Je größer die Streuung ist, desto geringer ist die Intensität der von dem Strahlungsempfänger 16 empfangenen Strahlung 15.

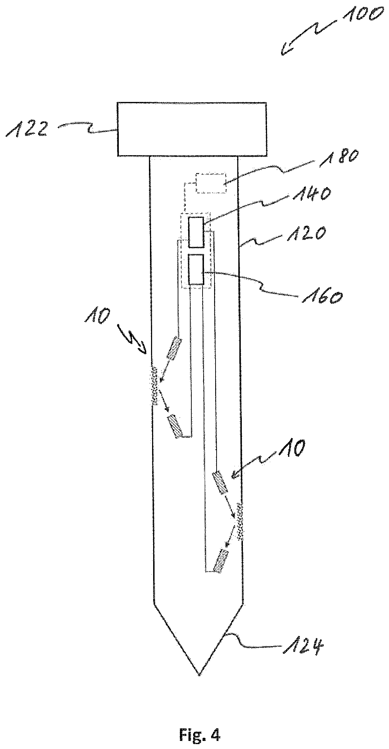

Im Folgenden wird im Zusammenhang mit Figur 4 eine erfindungsgemäße Messsonde 100 zum Messen von Matrixpotential in einem die Messsonde 100 umgebenden Medium 20 beschrieben.

Die Messsonde 100 umfasst einen Korpus 120. Der Korpus 120 kann beispielsweise eine zylindrische Form aufweisen. Die Messsonde 100 umfasst wenigstens einen erfindungsgemäßen optischen Sensor 10. Der wenigstens eine Sensor 10 ist derart in dem Korpus 120 angeordnet, dass das Messplättchen 12 durch den Korpus 120 hindurchragt. Genauer gesagt weist der Korpus 120 für jeden optischen Sensor 10 eine Aussparung auf, in der das jeweilige Messplättchen 12 derart angeordnet ist, dass seine Außenfläche 12b bündig mit einer Außenwand des Korpus 120 abschließt und somit mit dem zu messenden Medium 20 in Kontakt treten kann. Es versteht sich, dass ein Messplättchen 12 auch derart in dem Korpus 120 angeordnet sein können, dass seine Außenfläche 12b über die Außenwand des Korpus 120 hinausragt, oder dahinter zurückbleibt (d.h. eine Nische bildet).

Ferner umfasst die Messsonde eine Steuereinheit 140 und eine Auswerteeinheit 160. Die Steuereinheit ist dazu ausgebildet, die Strahlungsintensität und Strahlungsdauer der von dem Strahlungssender 14 emittierten Strahlung 15 zu steuern. Die Auswerteeinheit 160 ist dazu ausgebildet, das Matrixpotential in dem Medium anhand der Strahlungsintensität der von dem Strahlungsempfänger 16 empfangenen Strahlung 15 zu berechnen. Die Steuereinheit 140 und die Auswerteeinheit 160 können jeweils als Mikrocontroller implementiert sein. Es versteht sich, dass die Steuereinheit 140 und die Auswerteeinheit 160 auch als eine gemeinsame Rechnenkomponente ausgebildet sein können (angedeutet durch die gestrichelte Umrahmung der Komponenten 140 und 160 in Fig. 4).

Gemäß der Darstellung in Figur 4 umfasst die Messsonde 100 zwei erfindungsgemäße optische Sensoren 10. Die Sensoren 10 sind in Längsrichtung des Korpus 120 voneinander beabstandet angeordnet. Bei der Messung von Matrixpotential im Erdboden können auf diese Weise gleichzeitig Messungen in verschiedenen Tiefen vorgenommen werden. Somit kann die Effizienz und Aussagekraft von Messungen in Erdböden gegenüber Messungen in nur einem Tiefenbereich erheblich gesteigert werden. Bei dem Einsatz solcher Multilevel-Sonden können die verwendeten optischen Sensoren 10 eine zentrale Steuereinheit 140 und Auswerteeinheit 160 aufweisen, wie beispielhaft anhand der Darstellung in Figur 4 gezeigt. Die Steuereinheit 140 und die Auswerteeinheit 160 sind dann dazu ausgelegt, die jeweiligen Sensoren 10 zentral anzusteuern. Es versteht sich jedoch, dass jede der verwendeten optischen Sensoren 10 auch eine eigene unabhängige Steuereinheit 140 und Auswerteeinheit 160 aufweisen kann.

Die Messsonde 100 kann ferner ein Schnittstellenmodul 180 umfassen. Das Schnittstellenmodul 180 steht mit der Steuereinheit 140 und/oder der Auswerteeinheit 160 in Verbindung und ist dazu ausgebildet, mit einem externen Rechengerät (z.B. PC, Tablet, oder Smartphone) zu kommunizieren, von diesem z.B. Steuerbefehle zu empfangen und Sensordaten dorthin zu übermitteln.

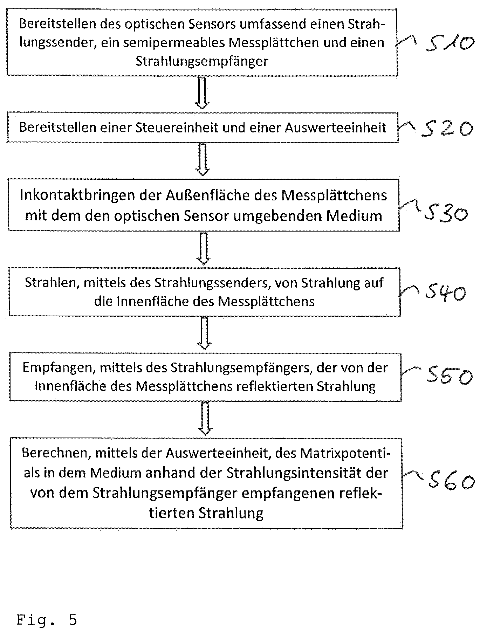

Figur 5 zeigt ein Flussdiagramm eines Verfahrens zum Messen eines Matrixpotentials in einem Medium mittels eines erfindungsgemäßen optischen Sensors, wobei der Sensor von dem Medium umgeben ist. Das Verfahren umfasst in einem ersten Schritt S10 das Bereitstellen des optischen Sensors mit dem Strahlungssender, dem semipermeablen Messplättchen und dem Strahlungsempfänger, sowie in einem zweiten Schritt S20 das Bereitstellen einer Steuereinheit und einer Auswerteeinheit. Weiter umfasst das Verfahren die folgenden Schritte S30 bis S60: Inkontaktbringen S30 der Außenfläche des Messplättchens mit dem den optischen Sensor umgebenden Medium; Strahlen S40, mittels des Strahlungssenders, von Strahlung auf die Innenfläche des Messplättchens, wobei die Strahlungsintensität und Strahlungsdauer der von dem Strahlungssender emittierten Strahlung durch die Steuereinheit gesteuert wird; Empfangen S50, mittels des Strahlungsempfängers, der von der Innenfläche des Messplättchens reflektierten Strahlung; und Berechnen S60, durch die Auswerteeinheit, des Matrixpotentials in dem Medium anhand der Strahlungsintensität der von dem Strahlungsempfänger empfangenen reflektierten Strahlung.

Der Schritt S50 des Empfangens der reflektierten Strahlung kann ferner das Erfassen einer Intensitätsänderung der empfangenen Strahlung umfassen, wobei die Intensitätsänderung auf eine Änderung des Matrixpotentials hinweist.

## Patentansprüche

1. Optischer Sensor (10) zur Erfassung von Strahlungsintensitäten, die auf ein Matrixpotential in einem den Sensor (10) umgebenden Medium (20) hinweisen, wobei der Sensor (10) umfasst:
ein semipermeables Messplättchen (12), das eine Innenfläche (12a) und eine Außenfläche (12b) aufweist, wobei die Außenfläche (12b) dazu ausgebildet ist, mit dem Medium (20) in Kontakt zu treten;
einen Strahlungssender (14), der dazu ausgebildet ist, Strahlung (15) auf die Innenfläche (12a) des Messplättchens (12) zu strahlen; und
einen Strahlungsempfänger (16), der dazu ausgebildet ist, die von der Innenfläche (12a) des Messplättchens (12) reflektierte Strahlung (15) zu empfangen;
**dadurch gekennzeichnet, dass** das Messplättchen (12) aus einem porösen photokatalytisch aktiven Material aufgebaut ist und die Strahlung (15) dazu konfiguriert ist, das photokatalytisch aktive Material anzuregen.

2. Optischer Sensor (10) nach Anspruch 1, wobei die Strahlung eine Wellenlänge im UV-Bereich aufweist.

3. Optischer Sensor (10) nach Anspruch 1 oder 2, wobei die Strahlung eine Wellenlänge zwischen 380 nm und 390 nm, insbesondere eine Wellenlänge von 385 nm aufweist.

4. Optischer Sensor (10) nach einem der vorhergehenden Ansprüche, wobei das Messplättchen (12) aus einer hydrophilen Keramik aufgebaut ist.

5. Optischer Sensor (10) nach einem der vorhergehenden Ansprüche, wobei das Messplättchen (12) aus Titandioxid aufgebaut ist.

6. Optischer Sensor (10) nach einem der vorhergehenden Ansprüche, wobei das Messplättchen (12) einen ersten Bereich aufweist, und wobei die Porengröße in dem ersten Bereich zwischen 400 nm und 800 nm liegt.

7. Optischer Sensor (10) nach einem der vorhergehenden Ansprüche, wobei das Messplättchen (12) einen zweiten Bereich aufweist, und wobei die Porengröße in dem zweiten Bereich unterhalb von 400 nm liegt.

8. Optischer Sensor (10) nach einem der vorhergehenden Ansprüche, wobei der Strahlungsempfänger (16) ferner dazu ausgebildet ist, eine Intensitätsänderung der empfangenen Strahlung (15) zu erfassen, wobei die Intensitätsänderung auf eine Änderung des Matrixpotentials hinweist.

9. Messsonde (100) zur Messung von Matrixpotential in einem die Messsonde (100) umgebenden Medium (20), wobei die Messsonde (100) umfasst:
einen Korpus (120);
wenigstens einen optischen Sensor (10) gemäß einem der Ansprüche 1 bis 8, wobei der wenigstens eine Sensor (10) derart in dem Korpus (120) angeordnet ist, dass das Messplättchen (12) wenigstens teilweise durch den Korpus (120) hindurchragt;
eine Steuereinheit (140), die dazu ausgebildet ist, die Strahlungsintensität und Strahlungsdauer der von dem Strahlungssender (14) emittierten Strahlung (15) zu steuern; und
eine Auswerteeinheit (160), die dazu ausgebildet ist, das Matrixpotential in dem Medium (20) anhand der Strahlungsintensität der von dem Strahlungsempfänger (16) empfangenen reflektierten Strahlung (15) zu berechnen.

10. Messsonde (100) nach Anspruch 9, wobei der Korpus (120) eine zylindrische Form aufweist und einen Durchmesser von weniger als 15 mm hat.

11. Messsonde (100) nach Anspruch 9 oder 10, wobei der Korpus (120) aus einem Kunststoff aufgebaut ist.

12. Messsonde (120) nach einem der Ansprüche 9 bis 11, wobei die Messsonde (100) wenigstens zwei der optischen Sensoren (10) umfasst, und wobei die wenigstens zwei optischen Sensoren (10) in Längsrichtung des Korpus (120) einen Abstand zueinander aufweisen.

13. Verfahren zum Messen, mittels eines optischen Sensors (12) gemäß einem der Ansprüche 1 bis 8, eines Matrixpotentials in einem den optischen Sensor (12) umgebenden Medium, das Verfahren umfassend die folgenden Schritte:
Bereitstellen des optischen Sensors (10) mit dem Strahlungssender (14), dem semipermeablen Messplättchen (12) und dem Strahlungsempfänger (16); ferner
Bereitstellen einer Steuereinheit (140) und einer Auswerteeinheit (160);
Inkontaktbringen der Außenfläche (12b) des Messplättchens (12) mit dem den optischen Sensor (12) umgebenden Medium;
Strahlen, mittels des Strahlungssenders (14), von Strahlung (15) auf die Innenfläche (12a) des Messplättchens (12), wobei die Strahlungsintensität und Strahlungsdauer der von dem Strahlungssender (14) emittierten Strahlung (15) durch die Steuereinheit (140) gesteuert wird;
Empfangen, mittels des Strahlungsempfängers (16), der von der Innenfläche (12a) des Messplättchens (12) reflektierten Strahlung (15); und
Berechnen, durch die Auswerteeinheit (160), des Matrixpotentials in dem Medium (20) anhand der Strahlungsintensität der von dem Strahlungsempfänger (16) empfangenen reflektierten Strahlung (15).

14. Verwendung eines photokatalytisch aktiven Materials und einer das photokatalytische Material anregenden Strahlung zur Messung eines Matrixpotentials.
